# EUROPEAN PATENT SPECIFICATION

(11) **EP 2 019 861 B1**
(45) Date of publication and mention of the grant of the patent: **07.09.2011**
(21) Application number: 07728587.2
(22) Date of filing: 27.04.2007
(51) Int. Cl.: C12N 5/071, A61K 35/23, A61P 13/12

(54) **KIDNEY-DERIVED STEM CELL POPULATION, IDENTIFICATION AND THERAPEUTIC USE**
AUS NIEREN GEWONNENE STAMMZELLENPOPULATION, IDENTIFIZIERUNG UND THERAPEUTISCHE VERWENDUNG
POPULATION DE CELLULES SOUCHES RÉNALES, IDENTIFICATION ET UTILISATION THÉRAPEUTIQUE

(30) Priority: 28.04.2006 IT FI20060099
(43) Date of publication of application: 04.02.2009
(73) Proprietor: Azienda Ospedaliero-Universitaria Careggi, 50141 Firenze (IT)
(72) Inventor: ROMAGNANI, Paola, I-50139 Firenze (IT); MAGGI, Enrico, I-50139 Firenze (IT); ROMAGNANI, Sergio, I-50139 Firenze (IT)
(74) Representative: Gervasi, Gemma
(86) International application number: PCT/EP2007/054132
(87) International publication number: WO 2007/125088

(56) References cited:
- WO-A-2005/108981
- BUSSOLATI B ET AL: "Isolation of renal progenitor cells from adult human kidney." THE AMERICAN JOURNAL OF PATHOLOGY, vol. 166, no. 2, February 2005 (2005-02), pages 545-555, XP002454238 ISSN: 0002-9440
- CANTLEY L G: "Adult stem cells in the repair of the injured renal tubule." NATURE CLINICAL PRACTICE. NEPHROLOGY, vol. 1, no. 1, November 2005 (2005-11), pages 22-32, XP008084383 ISSN: 1745-8323
- CHALLEN G A ET AL: "Identifying the molecular phenotype of renal progenitor cells." JOURNAL OF THE AMERICAN SOCIETY OF NEPHROLOGY, vol. 15, no. 9, September 2004 (2004-09), pages 2344-2357, XP002454239 ISSN: 1046-6673
- UCHIDA N ET AL: "Direct isolation of human central nervous system stem cells." PROCEEDINGS OF THE NATIONAL ACADEMY OF SCIENCES OF THE UNITED STATES OF AMERICA, vol. 97, no. 26, 19 December 2000 (2000-12-19), pages 14720-14725, XP002223508 ISSN: 0027-8424
- SAGRINATI C ET AL: "Isolation and characterization of multipotent progenitor cells from the Bowman's capsule of adult human kidneys." JOURNAL OF THE AMERICAN SOCIETY OF NEPHROLOGY, vol. 17, no. 9, September 2006 (2006-09), pages 2443-2456, XP002454240 ISSN: 1046-6673

## Description

### Field of the invention

The present invention relates to the field of stem cells and their use.

### State of the art

It is well known that many adult organs contain pluripotent stems cells involved in maintenance of tissue integrity and in repair processes.

The availability of kidney stem cells capable of regenerating kidney tissue after damage is very important for the prospect of prevention and therapy of any type of renal damage. In fact, acute and chronic kidney diseases represent a public health emergency because of their epidemiological relevance and of high costs involved in substitutive treatments, such as dialysis and transplant.

### Summary of the invention

The invention makes available kidney stem cells capable of co-expressing in that CD 133 and CD 24 markers on their surface

### Brief description of the figures

Figure 1 shows coexpression of CD24 and CD133 stem cell markers and identifies a cellular subpopulation in the Bowman's capsule of human adult kidneys.
Figure 2 relates to the isolation and characterization of CD24+CD133+ cells.
Figure 3 shows the proliferative capacity of CD24+CD133+ cells compared to CD24-CD133- cells.
Figure 4 shows the differentiation of clones obtained from CD24+CD133+ cells derived from the Bowman's capsule to tubular epithelial cells.
Figure 5 shows the differentiation of clones obtained from CD24+CD133+ cells derived from the Bowman's capsule to osteoblasts and adipocytes.
Figure 6 shows the acquisition of neuronal phenotypic and functional properties by clones obtained from CD24+CD133+ cells derived from the Bowman's capsule.
Figure 7 shows human kidney CD24+CD133+ cells implanted into kidneys of SCID mice affected by acute renal failure (ARF), and the generation of different types of tubular cells.
Figure 8 shows that CD24+CD133+ cells protect glycerol-treated mice from deterioration of kidney structure and function.

### Detailed description of the invention.

The present invention allows to overcome the above-mentioned problem by making available pluripotent kidney stem cells.

In fact, it has been surprisingly found that renal cells capable of co-expressing CD133 and CD24 markers (CD24+CD133+) on their surface possess stem cell capacity.

### Identification and isolation of kidney stem cells

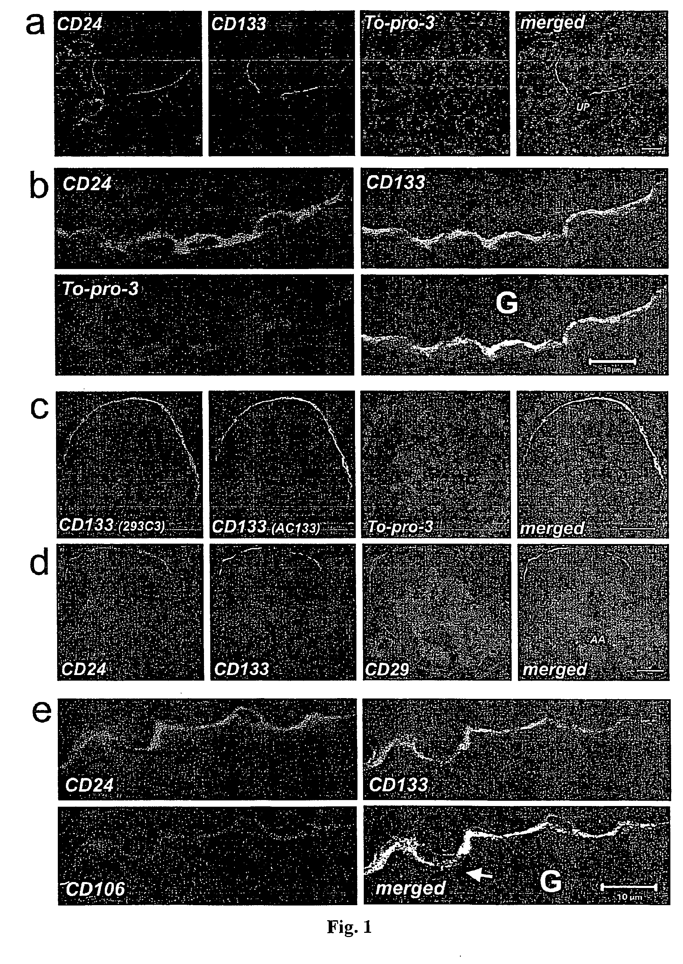

Twenty normal human kidneys have been examined by confocal microscopy using anti-CD24 and anti-CD133 monoclonal antibodies. CD24, a marker for the population of kideny embryonic progenitor cells, was found to be selectively expressed on cells of the parietal epithelium of the Bowman's capsule and in a subpopulation of tubular epithelial cells. The anti-CD133 monoclonal antibody, that selectively identifies stem cells derived from various human tissues, detected a subpopulation of Bowman's capsule cells and rare tubular structures (Fig. 1). Double immunofluorescence for CD24 and CD133 showed that the two markers identify a subpopulation of Bowman's capsule cells that is mostly located at level of the urinary pole of the glomerulus and often extends to the portion of the tubule closest to the urinary pole (Fig. 1). The same cells were also labelled with anti-CD106 monoclonal antibody. Overall, these results suggest the existence, in the adult human kidney, of a cell population at the level of Bowman's capsule epithelium and of a rare population of tubular cells, in which different stem cell markers are co-expressed, as shown in Figure 1, the various images of which are hereunder described in detail:
a) Double immunofluorescence staining, showing expression of CD24 (red) and CD133 (green) in Bowman's capsule cells from a kidney of a human adult subject. Superimposition of the two staining patterns (yellow) shows CD24 and CD133 coexpression in a subpopulation of cells localized in the urinary pole (UP, Bar 50 µm). To-pro-3 counterstains nuclei (blue).
b) Double immunofluorescence staining, detected at higher magnification, showing expression of CD24 (red) and CD133 (green) in Bowman's capsule cells. Superimposition of the two staining patterns shows CD24 and CD133 colocalization in the cytoplasm and on the membrane of cells facing the glomerulus (G) while only CD24 is expressed on the basal membrane. (yellow, Bar 10 µm). To-pro-3 counterstains nuclei (blue).
c) Detection of CD133 with two different anti-CD133 monoclonal antibodies. Antibody 293C3 (red) and antibody AC133 (green) stain the same subpopulation of cells in the Bowman's capsule. The superimposed image, showing both staining patterns, shows co-staining of the same cells (yellow, Bar 50 µm). To-pro-3 counterstains nuclei (blue).
d) Detection of CD24 (red), CD133 (green) and CD29 (blue) at the level of glomeruli. CD29 staining allows identification of the afferent arteriole (AA). The superimposed image shows that CD24 and CD133 are selectively coexpressed in a subpopulation of cells localized on the opposite side of the vascular pole (yellow, Bar 50 µm).
e) Triple immunofluorescence staining, detected at higher magnification, showing expression of CD24 (red), CD133 (green) and CD106 (blue) in capsule cells. The superimposed image shows colocalization of CD24 and CD133 (yellow) in the cytoplasm and on membrane of cells facing the glomerulus (G), while CD24 and CD106 (purple color) are co-expressed on the basal membrane. Visibile areas of CD24, CD133 and CD106 co-expression are stained in white (Bar 10 µm).

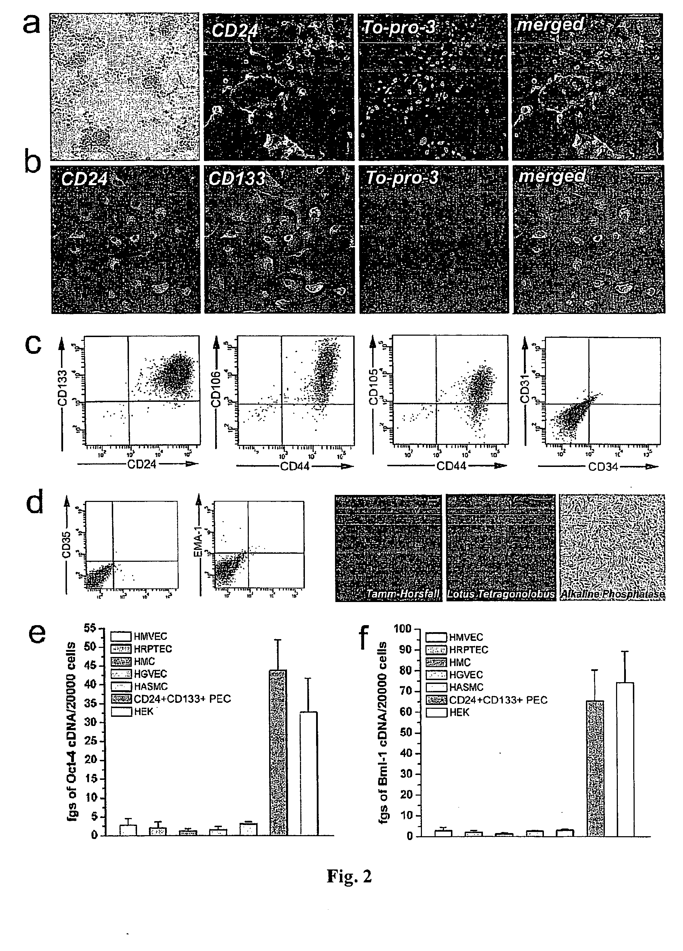

CD24+CD133+ cells were then isolated in order to evaluate their morphological and functional characteristics. For this purpose, the cortical component of kidney tissue was separated from the medullary component and subjected to crushing. Glomeruli were isolated by a standard separation technique using sieves of different porosity. To avoid destruction of Bowman's capsule CD24+CD133+ cells, the glomerular suspension was not digested but was directly cultured in plastic dishes containing EGM®-MV (Lonza Group, Walkersville, USA) supplemented with 20% FBS. Cells coming out from cultured glomeruli were examined for their ability to form cellular aggregates resembling neurospheres and their stem cell phenotype was assessed by confocal microscopy and cytofluorimetric analysis as shown in Figure 2, the various images of which are hereunder described in detail:
a) The first panel on the left shows proliferating cells coming out from a capsulated glomerulus in culture (x40). Subsequent images obtained by laser confocal microscopy show that the proliferating population expresses CD24 (green). To-pro-3 counterstains nuclei (blue). The superimposed image shows that proliferating cells express remarkable amounts of CD24 (Bar 100 µm).
a) Double immunofluorescence staining detecting the expression of CD24 (red) and CD133 (green), showing selective co-localization of the two markers in the population derived from proliferating glomerular cells. To-pro-3 counterstains nuclei (blue). The superimposed image shows intense CD24 and CD133 staining in the same cells (yellow, Bar 100 µm).
c) Primary culture of Bowman's capsule cells represent a homogeneous population comprising approximately 100% of cells expressing CD24, CD133, CD106, CD105 and CD44, and test negative for CD31 and CD34 endothelial cell markers. An analysis by flow cytometry of a representative culture is also shown.
d) A primary culture derived from CD24+CD133+ Bowman's capsule cells does not express kidney lineage markers, such as CD35 or EMA-1, as shown by flow cytometry in the first two panels. It is shown, by confocal microscopy, that the same cells do not express THG (third panel) and LTA (fourth panel) (Bar 100 µm). Negative histochemical staining for alkaline phosphatase (last panel). A representative culture is shown.
e) Measurement of Oct-4 mRNA levels by "Real Time quantitative RT-PCR" in cultures of endothelial cells, renal tubular cells, mesangial cells, podocytes, smooth muscle cells, CD24+CD133+ cells and HEK cells. Results are expressed as mean±SD of triplicate measurements performed on primary cultures from 5 different donors.
e) Measurement of Bml-1 mRNA levels by "Real Time quantitative RT-PCR" in cultures of endothelial cells, renal tubular cells, mesangial cells, podocytes, smooth muscle cells, CD24+CD133+ cells and HEK cells. Results are expressed as mean±SD of triplicate measurements performed on primary cultures from 5 different donors.

Isolated cells expressed both CD24 and CD133, while did not express any haematopoietic (CD34, CD45), endothelial (CD31, CD34), podocyte or tubular (EMA-1, Lotus Tetragonolobus, Dolichos Biflorus, alkaline phosphatase) marker (Fig. 2).

To better define the stem cell features of isolated cells, the expression of Oct-4, a typical embryonic stems cell marker, was evaluated together with the expression of Bml-1, another transcription factor which is critical for maintenance of the self-renewal ability of stem cells and for prevention of cellular senescence, thus demonstrating the stem cell nature of CD24+CD133+ cells (Fig. 2). This is also the first description of a population of kideny-derived cells expressing stem cell transcription factors Oct-4 and Bml-1 in culture.

To evaluate whether CD24+CD133+ cells also exhibit functional properties of stem cells, said cells were labelled in culture with CFDA-SE and plated in EGM®-MV supplemented with 20% FBS. The data on proliferation showed a much higher proliferative capacity of said cells compared to other renal cells not expressing the combination of CD133 and CD24 markers.

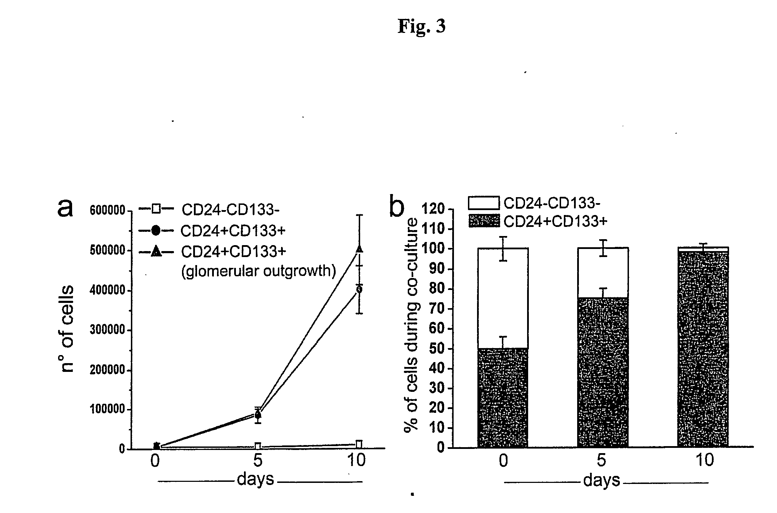

In fact, CD24-CD133- cells (i.e. not expressing CD133 and CD24) were prepared from adult kidney cortical tissue digested with enzymatic methods (for instance collagenase) to degrade the connective tissue, followed by separation by immunological methods, in particular immunomagnetic methods. These cells were then plated in the same media and, after adhesion, they were evaluated by cytofluorimetric analysis. When CD24+CD133+ cells were plated with CD24-CD133- cells in the same culture dishes in a 1:1 ratio, and in different media, the ratio between CD24+CD133+ and CD24-CD133- cells changed to approximately 9 to 1 after only 10 days of culture (Fig. 3). To validate further the evidence that CD24+CD133+ cell cultures exhibit functional properties characteristic of stem cells, said cells coming out from glomeruli in culture were detached and single cells were cloned by limiting dilution in "multi-well" plates coated with fibronectin. CD24-CD133- cells, obtained by immunomagnetic separation and also cloned by limiting dilution in "multi-well" plates coated with fibronectin, were used as control; only wells containing a single cell were evaluated. The clonogenic potential was found to be 41.3 ± 14% for CD24+CD133+ cells obtained from glomerular cultures and 2.1 ± 1.9 for CD24-CD133- cells. It should be noted that the rare clones derived from CD24-CD133- cells resulted from a small background contamination with CD24+CD133+ cells.

To assess the ability of cultured CD24+CD133+ cells to differentiate in various cell types, individual CD24+CD133+ cell clones from different donors were cultured under conditions favouring tubulogenic, adipogenic, osteogenic and neurogenic differentiation.

Tubulogenic differentiation was obtained by incubating CD24+CD133+ cell clones for 30 days in REBM commercial culture medium, containing SingleQuotes (hydrocortisone, hEGF, FBS, epinephrine, insulin, triiodothyronine, transferrin and gentamicin/amphotericin-B) (Cambrex Bio Science), supplemented with 50 ng/ml HGF (Peprotech, Rocky Hill, NJ).

Osteogenic, adipogenic and neurogenic differentiation of CD24+CD133+ cell clones was induced as previously described. For osteogenic induction, PEC CD24+CD133+ cells were cultured in α-MEM medium supplemented with 10% horse serum, containing in addition 100 nM dexamethasone, 50 µM ascorbic acid and 2 mM di β-glycerophosphate (all these reagents were from Sigma-Aldrich). The culture medium was replaced twice a week for 3 weeks. For adipogenic differentiation, CD24+CD133+ cells were incubated in DMEM high glucose (hg) (Invitrogen, Carlsbad, CA, USA) containing 10% di Fetal Bovine Serum (FBS), 1 µM di dexamethasone, 0.5 µM 1-methyl-3-isobutylxanthine (IBMX), 10 µg/ml insulin and 100 µM indometacin (all these reagents were from Sigma-Aldrich). After 72 hours, the medium was changed to DMEM hg with 10% FBS, containing 10 µg/ml insulin, for 24 hours. These treatments were repeated three times. Cells were then maintained in DMEM hg with 10% FBS and 10 µg/ml insulin for one additional week. For neurogenic differentiation, CD24+CD133+ cells were plated in DMEM hg with 10% FBS. After 24 hours, the culture medium was replaced with DMEM hg with 10% FBS, containing B27 (Invitrogen), 10 ng/ml di EGF (Peprotech) and 20 ng/ml bFGF (Peprotech). Five days later, cells were washed and incubated in DMEM supplemented with 5 µg/ml insulin, 200 µM indometacin and 0.5 mM IBMX, in absence of FBS, for 5 hours. Alizarin red, Oil-Red O or alkaline phosphatase staining was performed as described [see Romagnani P. et al: CD14+CD34low cells with stem cell phenotypic and functional features are the major source of circulating endothelial progenitors. Circ Res 97: 314-322, 2005; Boquest AC et al.: Isolation and transcription profiling of purified uncultured human stromal stem cells: alteration of gene expression after in vitro cell culture. Mol Biol Cell 16: 1131-1141, 2005; Pittenger MF et al.: Multilineage potential of adult human mesenchymal stem cells. Science 284: 143-147, 1999.

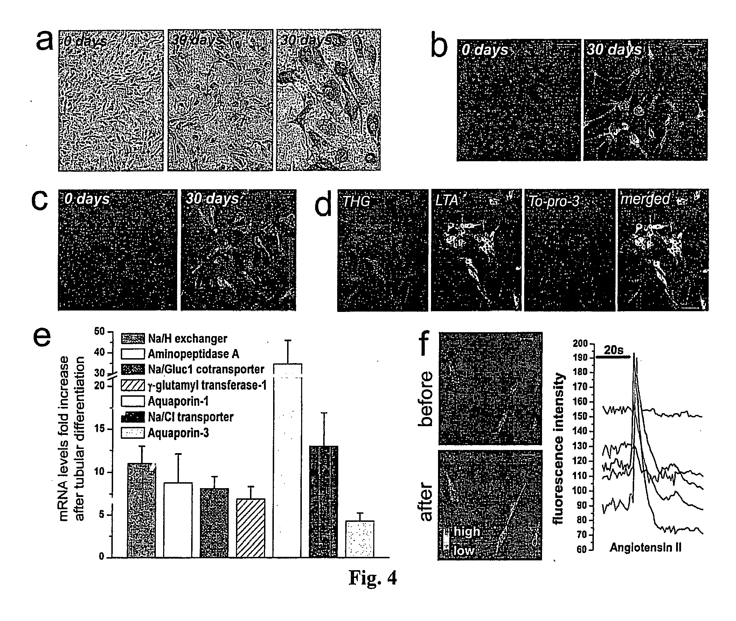

Tubulogenic differentiation has been demonstrated based on the assessment of acquisition of markers characteristic of fully differentiated kidney tubular epithelial cells, such as alkaline phosphatase, aminopeptidase A (primarily expressed by epithelial cells of the proximal tubule), Thiazide-Sensitive Na-Cl Cotransporter (primarily expressed by epithelial cells of the distal tubules), EMA-1, Lotus Tetragonolobus, Dolichos Biflorus and aquaporins 1 and 3. These markers were detected by confocal microscopy, cytofluorimetric analysis and mRNA determination by quantitative RT-PCR (Fig. 4).

Results are shown in Fig. 4, the various images of which are hereunder described in detail:
a) Representative photomicrographs of alkaline phosphatase histochemical staining of CD24+CD133+ cells before (day 0) and after (day 30) incubation in culture medium favouring tubular differentiation. Original magnification: x65, x80 and x320, respectively.
b) LTA staining before (day 0) and after (day 30) incubation in culture medium favouring tubular differentiation, as assessed by confocal microscopy (green). To-pro-3 blue staining counterstains nuclei (Bar 100 µm).
c) THG expression before (day 0) and after (day 30) incubation in culture medium favouring tubular differentiation, as assessed by confocal microscopy (green). To-pro-3 blue staining counterstains nuclei (Bar 100 µm).
d) LTA (green) and THG (red) double staining showing the coexistence in the same clone of cells co-expressing markers of different tubular segments (superimposed image, yellow) and cells expressing either proximal or distal tubular markers (Bar 100 µm).
e) Measurement by RT-PCR of increased mRNA levels for tubular markers after 30 days of culture in medium favouring tubular differentiation, compared to values obtained with the same cells prior to differentiation. Columns represent mean values±SD obtained after differentiation of 50 different clones.
f) Left: photomicrographs representative of confocal fluorescence images. Images recorded at 488nm excitation wavelength, before and after addition of angiotensin

II (1 µM) (Bar 20 µm). Right: time kinetics of changes in fluorescence intensity recorded in 5 individual cells of each of 10 different clones examined.

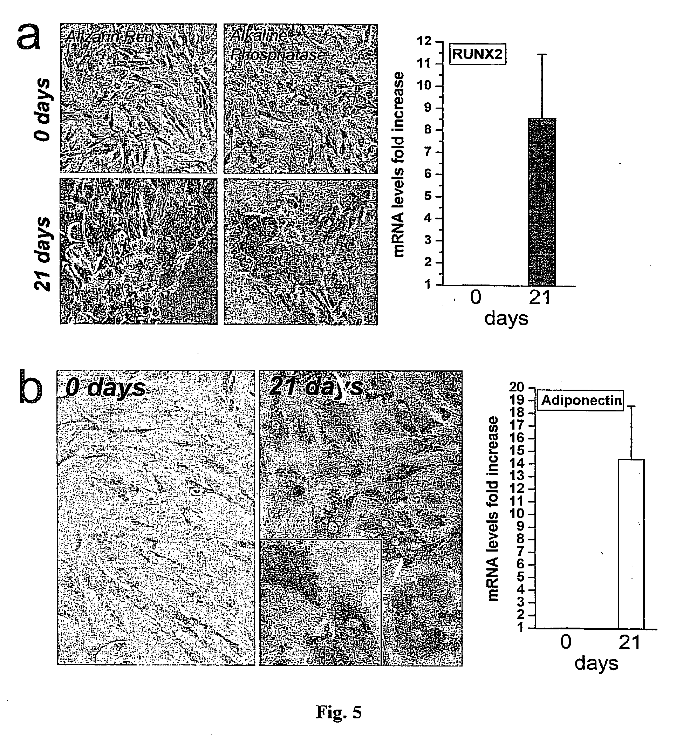

Adipogenic differentiation was demonstrated by assessing acquisition of the characteristic cellular morphology and from positive staining of lipid vacuoles with Oil-Red O. Moreover, quantitative RT-PCR showed a sharp increase of adiponectin mRNA levels (Fig. 5). Osteogenic differentiation was evaluated from the ability of cell clones to form alkaline phosphatase positive colonies; in the course of differentiation these colonies turned into mineralized nodules, as shown by Alizarin red staining that detects cellular calcium-rich deposits. Osteogenesis was further proved by the analysis of Runx2 mRNA expression. Results obtained following adipogenic and osteogenic differentiation are shown in Fig. 5, the various images of which are hereunder described in detail:
aaa) Left: representative photomicrographs of Alizarin and alkaline phosphatase histochemical staining before (day 0) and after (day 21) incubation of CD24+CD133+ cells in culture medium favouring osteogenic differentiation (x100). Right: measurement of Runx2 mRNA levels before (day 0) and after (day 21) incubation in the same culture medium. Columns represent mean values±SD obtained from 50 different clones.
b) Left: representative photomicrographs of Oil red-O histochemical staining before (day 0) and after (day 21) incubation of CD24+CD133+ cells in culture medium favouring adipogenic differentiation (x200). Inset: A few differentiated cells examined at higher magnification (x320). Right: measurement of Adiponectin mRNA levels on day 0 and after 21 days of incubation in the same culture medium. Columns represent mean values±SD obtained from 50 different clones.

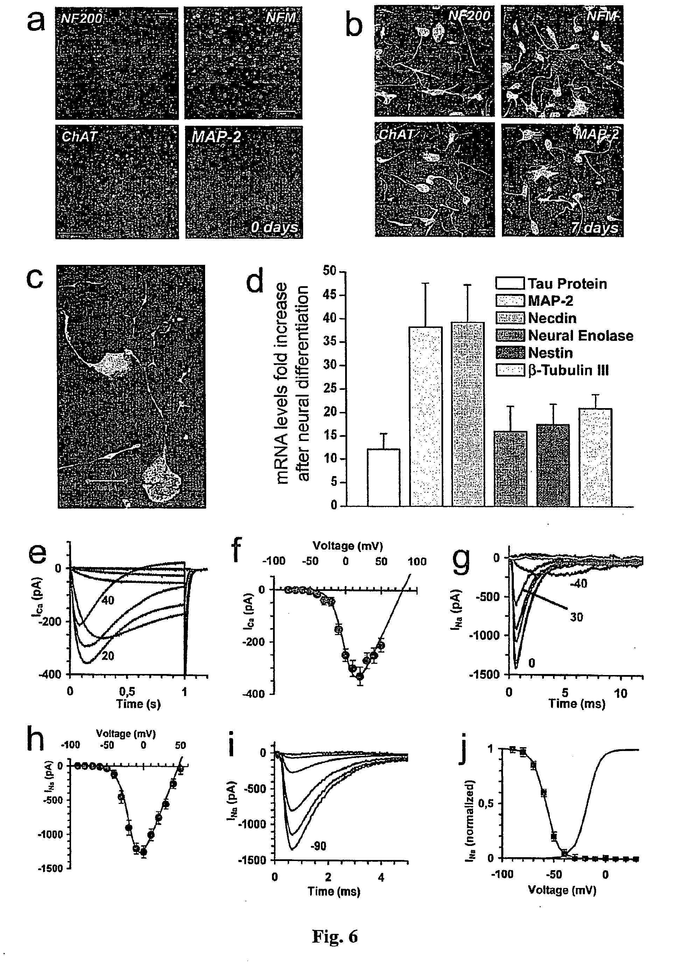

Neurogenic differentiation was demonstrated based on acquisistion of neuron-like morphology and high expression, at mRNA and protein levels, of tau protein, Microtubule Associated Protein (MAP-2), neuronal specific enolase, nestin, cholesterol-acetyl transferase, beta-tubulin III and neurofilament 200. Furthermore, electrophysiological studies made possible to demonstrate that the so obtained neuronal cells showed the presence of voltage dependent calcium and sodium channels with fully neuron-like characteristics, as shown in Fig. 6, the various images of which are hereunder described in detail:
a) Absence of NF200, NFM, ChAT and MAP-2 neuronal markers before incubating CD24+CD133+ cells in medium favouring neurogenic differentiation, assessed by confocal microscopy. To-pro-3 counterstains nuclei (Bar 100 µm). A representative experiment is shown.
b) Strong expression of NF200, NFM, ChAT and MAP-2 neuronal markers after differentiation of cells in the same medium (green). To-pro-3 counterstains nuclei (Bar 100 µm). A representative experiment is shown.
c) Higher magnification of a representative experiment, showing acquisition of typical neuronal morphology, as well as ChAT staining (green) in CD24+CD133+ cells cultured under conditions favouring neurogenic differentiation (Bar 100 µm).
d) Measurement by "Real-time quantitative RT-PCR" of increased mRNA levels for different neuronal markers after culturing cells under conditions favouring neurogenesis, compared to values from the same cells prior to neurogenic differentiation. Columns represent mean values ± SD obtained from 50 different clones.
e-h) Ca²⁺ and Na⁺ currents in neurons derived from CD24+CD133+ cells.

Representative currents recorded at a potential of -90 mV, impulses of 1-s have been applied on a scale of -80 to 50 mV with 10-mV increments. Data have been acquired at different sampling times (50 µs in the first 100 ms and 1 ms for the remaining duration of the test) in order to detect fast and slow phenomena and time kinetics of L type Ca²⁺ current I_{Ca}; for clarity, only current traces recorded at - 60, -40, -20, 0, 20, 30 and 40 mV are presented. f I_{Ca}-V curve determined at the current peak (n = 26). g Time kinetics of the Na⁺ current I_{Na}; only current traces recorded at -60, -40, -30, -20, -10, 0, 20 and 30 mV are presented; the red line indicates I_{Na} induced at 0 mV in presence of TTX (1 µM). h I_{Na}-V curve determined at the current peak (n = 26). f, h The continuous line superimposed on the data represents the activation function: *Iₐ*(*V*)=*G*ₘₐₓ(*V-Vᵣₑᵥ*)/{1+exp[(*Vₐ-V*)/*kₐ*]}, where *G*ₘₐₓ is the maximum conductance, *V*ᵣₑᵥ is the apparent inversion of potential, *V*ₐ is the potential that induces half maximum increase of conductance and *k*ₐ is the slope factor. i Inactivation of I_{Na} evoked at a potential of -90 mV; only traces without pre-impulse (-90 mV) and with -70, -60, -30, -40 e -30 mV pre-impulse are presented. j Inactivation curve normalized for I_{Na}, the continuous line superimposed on the data represents the inactivation function: *Iₕ*(*V*)=1/{1+exp[-(*Vₕ*-*V*)/*kₕ*]}, where *V*ₕ is the potential triggering the half-maximal current and *k*ₕ is the slope factor for the inactivation. For comparison, the curve reported on the right relates to the activation.

According to the invention, CD24+CD133+ cells were also isolated by purification with immunomagnetic separation from total kidney tissue digested with collagenase, and the same was done also for CD24-CD133- cells.

In this case, cells of interest were obtained by purification of the cell suspension, obtained as described above, for CD133 and then for CD24 or viceversa. This way, it is possible to obtain virtually all CD24+CD133+ cells. The population obtained by these various method consists of a mixed population of Bowman's capsule cells and of cells derived from rare kidney tubules expressing CD133 and CD24. Overall, the CD24+CD133+ cell population represents about 0.5-9% of all resident kidney cells, and varies among individuals. All the experiments described above were also repeated with a CD24+CD133+ population obtained with the various methods of immunomagnetic separation listed above, and confirmed that kidneys contain a population of adult stem cells endowed with regenerative and amplifying capacity, characterized by co-expression of CD133 and CD24 markers, derived mostly from the urinary pole of the Bowman's capsule and for a very minor part from renal tubular cells.

Moreover, the present invention relates to compositions for therapeutic use, containing kidney stem cells that are capable, as described above, of co-expressing CD133+ and CD24+ markers and endowed with tubulogenic, adipogenic, osteogenic and neurogenic regenerative capacity.

In addition, the invention relates to the use of said cells for preparation of compositions useful for repairing kidney damage.

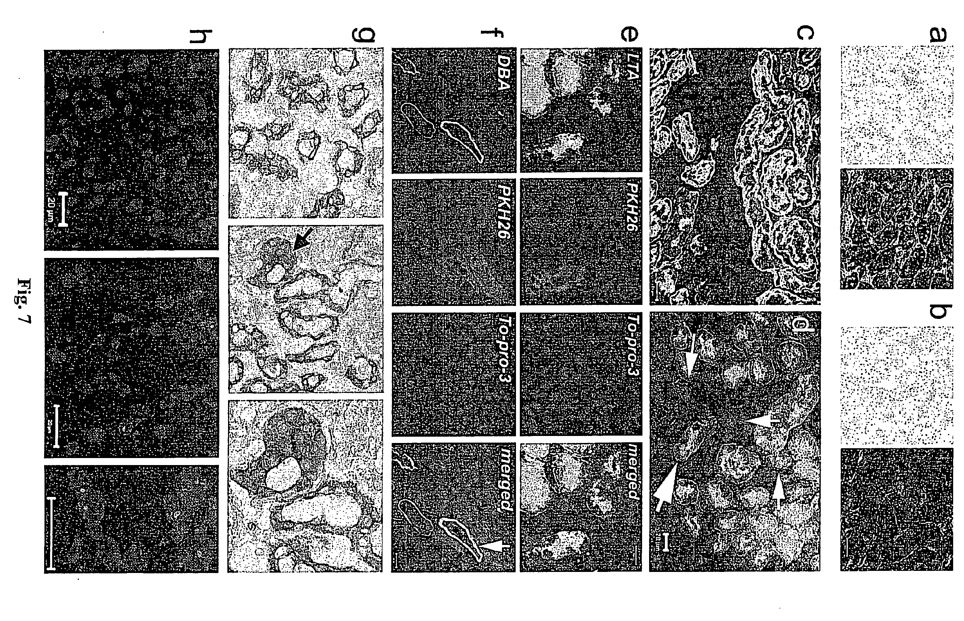

A model of acute tubular necrosis in SCID mice has been used to test whether the stem cells identified by the applicants, isolated from adult human kidney, can regenerate damaged kidney tissue. This model involves intramuscular injection of hypertonic glycerol that causes massive myolysis and hemolysis, resulting in severe tubular damage and acute renal failure. Tubular damage peaks at the third day after injection and then spontaneously regresses after about 10 days. The extent of induced damage was evaluated by hematoxylin/eosin staining, showing extensive necrosis of tubular epithelial cells, formation of hyaline tubular cylinders, loss of the brush border from proximal tubules and flattening of tubular epithelium. On the third and fourth day, a group of 8 mice was inoculated in the tail vein with 1.5 x 10⁶ CD24+CD133+ cells, while another group of 8 mice was inoculated with saline and a third group of 8 mice was inoculated with 1.5 x 10⁶ CD24-CD133-kidney cells, using the same procedure. The integration of CD24+CD133+ stem cells in damaged tubuli was demonstrated by use of cells labeled with the fluorescent dye PKH26 (red) and simultaneous labelling of proximal and distal tubules with LTA and "Dolicholus Biflorus Agglutinin", respectively. Furthermore, integration of the stem cell population in tubular structures was confirmed by an immunohistochemical technique that made use of the HLA-I antigen specific for human cells and cytokeratin as general epithelial cell marker. At last, the FISH method for Y chromosome was used to detect kideny cells from male human donors inoculated in female SCID mice. Results of all three methods showed selective integration of CD24+CD133+ cells in renal tubules of injected SCID mice. No integration could be shown in mice injected with saline or in those injected with CD24-CD133- human kideny cells, as shown in Figure 7, the various images of which are hereunder described in detail:
a) Classical microscopy showing kideny tissue of a normal mouse stained with hematoxylin/eosin (H&E, left) or with phalloidin (green, right) (Bar 50 µm).
b) Necrotic tubular damage observed after intramuscular injection of glycerol, as shown by H&E (left) or phalloidin (right) staining, with the latter showing loss of brush border and flattening of epithelial cells (green, Bar 50 µm).
c) Representative photomicrograph showing a kidney section from an acute renal failure SCID mouse injected with human CD24-CD133- kidney cells; LTA staining shows no red-stained cells by confocal microscopy (Bar 20 µm).
d) Representative photomicrograph showing a kidney section from acute renal failure mice injected with CD24+CD133+ cells treated with PKH26-label (red), stained with LTA (green), as shown by confocal microscopy. Small arrows indicate the presence of many red cell. The big arrow indicates a proximal tubule (Bar 20 µm).
e) Higher magnification of the kideny section in d) showing regeneration of a proximal tubular structure (Bar 20 µm).
f) Higher magnification of another kideny section obtained from an acute renal failure SCID mouse injected with PEC CD24+CD133+ cells treated with PKH26-label (red), and DBA-stained on the basal surface of two tubular structures (green), showing regeneration of a collector tubule structure (arrow). Other tubular structures stained by PKH26, but not the collector duct marker DBA, are visible. (Bar 20 µm).
g) Double immunohistochemical staining for cytokeratin (blue) and human class I HLA antigens (red) in kidneys of SCID mice with glycerol-induced acute renal failure (ARF). Left panel: absence of red signal in tubules of a kidney section from a mouse injected with CD24-CD133- cells; middle and right panels: positive cell staining for human class I HLA antigens (red, arrows) in cytokeratin expressing tubules (blue) from SCID mice with acute renal failure (ARF) induced by glycerol after injection of PEC CD24+CD133+ cells.
h) Y chromosome detection by FISH technique in control mice injected with saline (left panel) and in kidneys of female mice injected with CD24+CD133+ cells obtained from human male kidneys (red, middle and right panels) (Bar 20 µm). Identification of this population in the kidney, and demonstration of their repair capacity, has important implications in the field of regenerative medicine, for the therapy of renal pathologies.

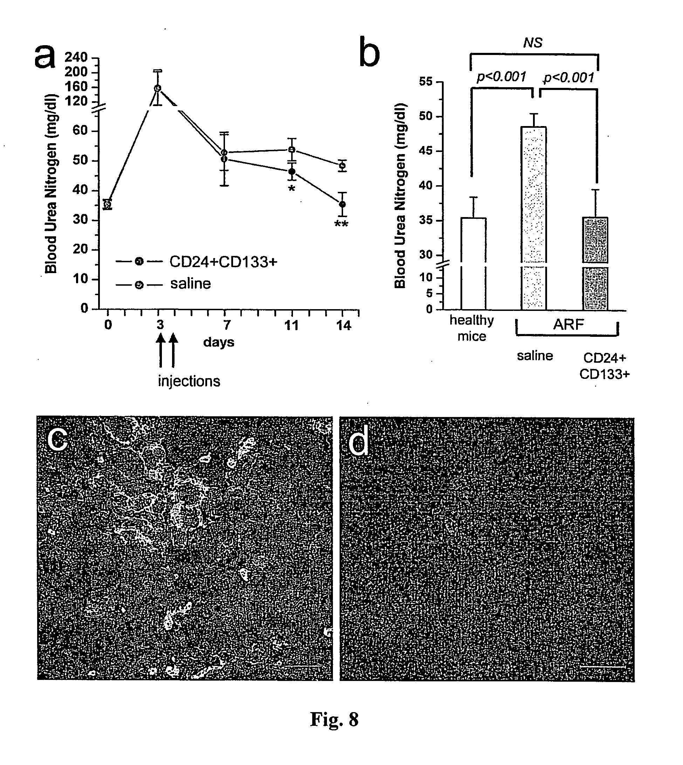

At last, the in vivo repair potential of the CD24+CD133+ pluripotent kidney stem cell population and consequent recovery of kidney functionality has been assessed by two distinct experimental approaches. In fact, azotemia was measured at various times following glycerol injection, both in mice treated with CD24+CD133+ cells and in those injected with saline. Compared to mice injected with saline, mice treated with CD24+CD133+ cells showed, 14 days after glycerol injection, significantly lower azotemia values that were fully comparable to values measured in the same mice prior to induction of renal damage. Moreover, the presence of fibrotic areas was evaluated by "alpha-SMA" staining in the same mice 14 days after glycerol injection. The group of mice subjected to injection of CD24+CD133+ cells showed significantly lower extension of fibrotic areas compared to the group of mice injected with saline, as shown in Figure 8, the various images of which are hereunder described in detail:
*a*. Blood nitrogen levels (BUN) were measured at various intervals in glycerol-treated mice that received saline (red line) or CD24+CD133+ cells (black line). Columns represent mean values±SD. *n* = 8 for each time point; total 80 mice. **p* < 0.01 and ***p* < 0.001 versus glycerol + saline at the same times.
*b*. Comparison of blood nitrogen levels (BUN) between healthy mice (white), mice treated with saline (light grey) and mice treated with CD24+CD133+ cells (dark grey) at day 14.
*c*. Representative photomicrograph of kidneys from mice treated with saline, stained for the presence of α -SMA (green). Nuclei are stained with To-pro-3 (Bar 100 µm).
*d*. Representative photomicrograph of kidneys from mice treated with CD24+CD133+ cells and stained for the presence of α -SMA (green). Nuclei are stained with To-pro-3 (Bar 100 µm).

## Claims

1. Kidney stem cells **characterized in that** they co-express CD133 and CD24.

2. Kidney cells according to claim 1 **characterised in that** they are originated from the Bowman's capsule

3. Composition comprising kidney stem cells according to claims 1 and 2.

4. Process for isolation of renal stem cells according to claims 1 and 2, wherein:
- renal glomeruli were isolated by a standard separation technique employing sieves with different permeability;
- the so obtained glomerular suspension was directly cultured in plastic dishes containing EGM®-MV supplemented with 20% FBS;
- cells coming out from glomeruli in culture and expressing CD133 and CD24 markers were isolated

5. Process for isolation of cells according to claims 1 - 2 by immunomagnetic separation from whole kidney tissue digested with collagenase, using CD133 and CD24 markers

6. Use of stem cell population according to claim 1-2 for the preparation of a composition for repair of kidney damage.

## Patentansprüche

1. Nierenstammzellen, **dadurch gekennzeichnet, dass** diese CD 133 und CD24 koexprimieren.

2. Nierenzellen nach Anspruch 1, **dadurch gekennzeichnet, dass** diese aus der Bowmannschen Kapsel stammen.

3. Zusammensetzung, welche Nierenstammzellen nach Anspruch 1 oder 2 enthält.

4. Verfahren zum Isolieren von Nierenstammzellen nach Anspruch 1 oder 2, wobei:
- Nierenglomeruli durch eine Standardtrenntechnik, bei welcher Siebe mit verschiedener Durchlässigkeit eingesetzt wurden, isoliert worden sind,
- die so erhaltenen Glomeruli enthaltende Suspension direkt in Plastikschalen kultiviert wurde, welche mit 20 % FBS ergänztes EGM®-MV enthielten,
- aus den Glomeruli in Kultur herauskommende und CD 133- und CD24-Marker exprimierende Zellen isoliert wurden.

5. Verfahren zum Isolieren von Zellen nach Anspruch 1 oder 2 durch immunomagnetische Trennung von Gesamtnierengewebe, welches mit Kollagenase verdaut wurde, unter Verwendung von CD 133- und CD24-Markern.

6. Verwendung einer Stammzellenpopulation nach Anspruch 1 oder 2 zur Herstellung einer Zusammensetzung zur Reparatur von Nierenschäden.

## Revendications

1. Cellules souches de rein **caractérisées en ce qu'**elles coexpriment CD133 et CD24.

2. Cellules de rein selon la revendication 1, **caractérisées en ce qu'**elles proviennent de la capsule de Bowman.

3. Composition comprenant des cellules souches de rein selon les revendications 1 et 2.

4. Procédé permettant d'isoler des cellules souches de rein selon les revendications 1 et 2, dans lequel :
- on a isolé des glomérules rénaux par une technique de séparation type employant des tamis de perméabilités différentes ;
- on a mis directement en culture la suspension glomérulaire ainsi obtenue dans des coupelles plastique contenant du EGM®-MV complété avec du FBS à 20 % ;
- on a isolé les cellules provenant de glomérules en culture et exprimant les marqueurs CD133 et CD24.

5. Procédé permettant d'isoler des cellules selon les revendications 1 à 2 par séparation immunomagnétique à partir d'un tissu rénal total digéré avec de la collagénase utilisant les marqueurs CD133 et CD24.

6. Utilisation d'une population de cellules souches selon les revendications 1 à 2 pour la préparation d'une composition destinée à réparer des lésions rénales.
